## Europäisches Patentamt

## European Patent Office

## Office européen des brevets

(19)

(11) Veröffentlichungsnummer: **0 351 627**

**A1**

(12) # EUROPÄISCHE PATENTANMELDUNG

(21) Anmeldenummer: 89112150.1

(22) Anmeldetag: 04.07.89

(51) Int. Cl.⁴: **C07C 63/04 , C07C 51/487 , C07C 51/44**

(30) Priorität: 16.07.88 DE 3824265

(43) Veröffentlichungstag der Anmeldung:
**24.01.90 Patentblatt 90/04**

(84) Benannte Vertragsstaaten:
**AT CH DE FR GB LI NL**

(71) Anmelder: **BAYER AG**

**D-5090 Leverkusen 1 Bayerwerk(DE)**

(72) Erfinder: **Schnatterer, Albert, Dr.**
**Walter-Flex-Strasse 32**
**D-5090 Leverkusen 1(DE)**
Erfinder: **Fiege, Helmut, Dr.**
**Walter-Flex-Strasse 23**
**D-5090 Leverkusen 1(DE)**
Erfinder: **Richter, Wolfgang, Dr.**
**Scheiblerstrasse 11**
**D-4150 Krefeld(DE)**

(54) **Verfahren zur destillativen Gewinnung von ortho-Tolylsäure.**

(57) o-Tolylsäure kann aus den bei der Sauerstoffoxidation von o-Xylol anfallenden Reaktionsgemischen destillativ gewonnen werden, wenn die die reine o-Tolylsäure enthaltende Destillatfraktion in einem Druckbereich von 70 bis 300 mbar entnommen wird und dem Destillationssumpf vorher eine Verbindung der Formel
$H_2N-R^1$
zugesetzt wurde, in der $R^1$ die in der Beschreibung genannte Bedeutung hat.

EP 0 351 627 A1

EP 0 351 627 A1

## Verfahren zur destillativen Gewinnung von o-Tolylsäure

Die Erfindung betrifft ein Verfahren zur destillativen Gewinnung von o-Tolylsäure aus den durch Oxidation von o-Xylol mit Sauerstoff erhaltenen Reaktionsgemischen.

Verfahren zur Herstellung von o-Tolylsäure durch Sauerstoffoxidation von o-Xylol in Gegenwart von Übergangsmetallkatalysatoren wurden mehrfach beschrieben (beispielsweise JP 56/156 232 (1981), JP 50/49 247 (1975)). Die Umsetzung wird meist in flüssiger Phase mit oder ohne Zusatz eines Lösungsmittels durchgeführt, wobei zur Vermeidung von Überoxidation die Reaktion nach einem Teilumsatz des o-Xylols abgebrochen wird. Die Reaktionsgemische enthalten in Abhängigkeit von der jeweiligen Verfahrensvariante in mehr oder weniger großem Umfang neben nicht umgesetztem o-Xylol die o-Tolylsäure, sowie teiloxidierte Verbindungen (beispielsweise o-Tolylalkohol und o-Tolylaldehyd), überoxidierte Verbindungen (beispielsweise Phthalid, 2-Carboxy-benzaldehyd, Phthalsäure und Phthalsäureanhydrid) und Produkte von Sekundärreaktionen (beispielsweise Phthalsäure-mono-o-tolyl ester und o-Tolylsäure-o-tolylester). Wird die Oxidation in einem Lösungsmittel durchgeführt, können zudem Produkte entstehen, denen eine Reaktion mit dem Lösungsmittel zugrundeliegt. Im Falle von Essigsäure als Lösungsmittel kann sich beispielsweise Essigsäure-o-tolylester bilden.

Die Vielfalt der Produkte erschwert die Reinisolierung der o-Tolylsäure erheblich. Zudem ist es wünschenswert, daß außer dem o-Xylol auch die teiloxidierten Verbindungen, beispielsweise o-Tolylalkohol und o-Tolylaldehyd, in einer Weise vom Reaktionsgemisch abgetrennt werden, daß eine Rückführung zur Oxidation möglich ist.

Die bekannten Verfahren zur Isolierung der o-Tolylsäure aus dem Reaktionsgemisch der o-Xyloloxidation benutzen als wesentliche Maßnahme die Kristallisation. In US 3 607 920 ist ein Verfahren beschrieben, wonach die o-Tolylsäure durch Kristallisation aus dem Reaktionsgemisch nach dessen Aufkonzentrierung abgetrennt wird. Nach JP 56/55 341 (1981) wird das Reaktionsgemisch nach Abdestillieren der Leichtsieder aus Xylol umkristallisiert. Nach zwei Kristallisationsvorgängen ist die o-Tolylsäure 99,2 %ig. Die Mutterlauge wird anschließend mit wäßrigem Alkali behandelt und der organische Anteil zur Oxidation zurückgeführt. Die Nachteile dieser Verfahren sind unbefriedigende Ausbeuten, aufwendige Verfahrensschritte und die Erzeugung von Abwässern, die mit Schwermetallen und organischem Material belastet sind.

In JP 56/156 232 (1981) ist ein weiteres Verfahren zur Herstellung von o-Tolylsäure durch kontinuierliche Oxidation von o-Xylol beschrieben. Die Aufarbeitung erfolgt destillativ, wobei eine o-Tolylsäure mit einem Gehalt von 99 % erhalten wird. Obwohl sich mit dem destillativen Aufarbeitungsverfahren die obengenannten Probleme prinzipiell umgehen lassen, ist dort keine für eine technische Realisierung brauchbare Lösung angeboten, die zu einer optimalen Aufarbeitung des Reaktionsgemisches der o-Xyloloxidation führt und damit eine möglichst quantitative Gewinnung der o-Tolylsäure und möglichst quantitative Rückführung von o-Tolylalkohol und o-Tolylaldehyd gewährleistet.

Es wurde nun gefunden, daß aus Reaktionsgemischen, welche bei der Sauerstoffoxidation von o-Xylol anfallen, die o-Tolylsäure in sehr hoher Ausbeute und Reinheit destillativ gewonnen werden kann, wenn die die reine o-Tolylsäure enthaltende Destillatfraktion in einem Druckbereich von 70 bis 300 mbar entnommen wird und dem Destillationssumpf vorher 1 bis 15 $NH_2$-Äquivalente pro Mol im Destillationssumpf vorhandener Phthalsäure oder Phthalsäurederivate einer oder mehrer Verbindungen der Formel

$H_2N-R^1$     (I)

zugesetzt wurde, worin
$R^1$ für H, Cl, Br, OH, CN, $NH_2$, $CONH_2$, $NHCONH_2$, $R^2$, $NHR^2$, $OR^2$, $COR^2$, $SO_2R^2$ oder $CONHR^2$ steht, wobei
$R^2$ geradkettiges oder verzweigtes $C_1$-$C_{30}$-Alkyl, $C_6$-$C_{12}$-Aryl oder $C_7$-$C_{10}$-Aralkyl bedeutet und wobei $R^2$ durch COOH, $C_1$-$C_4$-Alkoxy, OH und/oder $NH(C_2$-$C_{12}$-Alkylen-NH$)_n$-H substituiert sein kann, worin n eine Ziffer von 0 bis 8 bedeutet.

Als Einsatzgemische für die Destillation im erfindungsgemäßen Verfahren eignen sich Reaktionsgemische, die bei der Oxidation von o-Xylol zu o-Tolylsäure anfallen und neben der o-Tolylsäure noch weitere Verbindungen der obengenannten Art enthalten. Solche Reaktionsgemische können in flüssiger Phase oder in der Gasphase erhalten werden. Bevorzugt sind Gemische, welche bei der Sauerstoffoxidation von o-Xylol in flüssiger Phase mit oder ohne Lösungs- oder Verdünnungsmittel in Gegenwart katalytischer Mengen einer Schwermetallverbindung gebildet werden. Solche Schwermetallverbindungen können beispielsweise Verbindungen des Kobalts, Mangans, Eisens, Nickels, Chroms, Vanadiums, Kupfers, Silbers, Zirkons, Molybdäns usw. sein, bevorzugt Verbindungen des Kobalts, Mangans und/ oder Nickels. Lösungs- oder Verdünnungsmittel für die Oxidation können beispielsweise Kohlenwasserstoffe, chlorierte Kohlenwasserstoffe, aliphatische Carbonsäuren usw. sein. Die Oxidation kann aber auch in Gegenwart von Wasser in

2

einem 2-Phasen-System durchgeführt werden. Besonders bevorzugt werden auch Reaktionsgemische eingesetzt, die bei der Sauerstoffoxidation von o-Xylol zu o-Tolylsäure in niederen aliphatischen Carbonsäuren, beispielsweise Essigsäure als Lösungs- oder Verdünnungsmittel in Gegenwart katalytischer Mengen einer Schwermetallverbindung, beispielsweise einer Verbindung des Kobalts, Mangans usw. und einer katalytischen Menge eines Aktivators entstehen.

Solche Aktivatoren sind beispielsweise Bromverbindungen, wie ein Metallbromid oder eine organische Bromverbindung, oder Oxoverbindungen, wie Paraldehyd, Acetaldehyd, Methylethylketon usw.

Die erfindungsgemäße Gewinnung von o-Tolylsäure ist dadurch gekennzeichnet, daß die Gegenwart einer Verbindung der Formel (I) gearbeitet wird.

Als geradkettiges oder verzweigtes $C_1$-$C_{30}$-Alkyl sei beispielsweise Methyl, Ethyl, Propyl, Isopropyl, Butyl, Isobutyl, tert.-Butyl, die isomeren Amyle, Hexyle, Octyle, Decyle oder Dodecyle genannt. In bevorzugter Weise sei $C_1$-$C_{20}$-Alkyl genannt, in besonders bevorzugter Weise $C_1$-$C_{12}$-Alkyl.

Als $C_6$-$C_{12}$-Aryl sei beispielsweise Phenyl, Naphthyl oder Biphenylyl, bevorzugt Phenyl, genannt.

Als $C_7$-$C_{10}$-Aralkyl sei beispielsweise Benzyl, 1-Phenyl-ethyl, 2-Phenyl-ethyl, 1-, 2- oder 3-Phenyl-propyl, bevorzugt Benzyl, genannt.

Die genannten geradkettigen oder verzweigten Alkylreste, die Arylreste und die Aralkylreste können ihrerseits durch COOH, $C_1$-$C_4$-Alkoxy, wie Methoxy, Ethoxy, Propoxy, Isopropoxy, Butoxy oder Isobutoxy, durch OH und/oder durch $NH(C_2$-$C_{12}$-Alkylen-$NH)_n$-H substituiert sein. $C_2$-$C_{12}$-Alkylen ist hierbei beispielsweise 1,2-Ethylen, 1,2- oder 1,3-Propylen, 1,2-, 1,3- oder 1,4-Butylen, Hexylene, Octylene, Decylene oder Dodecylene, die geradkettig oder verzweigt sein können und deren Bindungen an das außerhalb und an das innerhalb der runden Klammer stehende N-Atom sich in 1,2-, 1,3- oder $\alpha,\omega$-Positition solcher Alkylengruppen befinden. Der Index n hat die Bedeutung einer Ziffer von 0 bis 8, bevorzugt 0 bis 6, besonders bevorzugt 0 bis 4. Durch diese zuletztgenannte Substitution fallen unter die Formel (I) beispielsweise Aminocarbonsäuren, Etheramine, Aminoalkohole oder Polyamine. Weitere Stoffe, die unter die Formel (I) fallen sind primäre Amine aliphatischer, aromatischer oder araliphatischer Art. Des weiteren fallen unter die Formel (I) Ammoniak, Chloramin, Bromamin, Hydroxylamin, Cyanamid, Hydrazin, Harnstoff, Semicarbazid, substituierte Hydrazine, Hydroxylaminether, Carbonsäureamide, Sulfonsäureamide und substituierte Harnstoffe.

Die Stoffe der Formel (I) können dem Destillationssumpf des zu destillierenden Gemisches als solche zugesetzt werden oder in Form von Verbindungen, aus denen sich die Stoffe der Formel (I) bilden, beispielsweise aus den Salzen von $H_2N$-$R^1$, beispielsweise ihren Hydrochloriden, aus den zugehörigen Iminen oder den zugehörigen Imiden.

In bevorzugter Weise werden Stoffe der Formel

$H_2N$-$R^{11}$   (II)

eingesetzt, in denen

$R^{11}$ für H, OH, $NH_2$, $CONH_2$, $NHCONH_2$, $R^2$, $COR^2$ oder $CONHR^2$ steht, wobei
$R^2$ die oben genannte Bedeutung hat.

In besonders bevorzugter Weise werden Stoffe der Formel $H_2N$-$R^{21}$   (III)
eingesetzt, in denen

$R^{21}$ für Wasserstoff, geradkettiges oder verzweigtes $C_1$-$C_{20}$-Alkyl, Phenyl, Benzyl oder -($C_2$-$C_{12}$-Alkylen-$NH)_m$-H steht, worin m eine Ziffer von 1 bis 8 bedeutet COOH, $C_1$-$C_4$-Alkoxy und/oder OH substituiert sein können.

Unter den Stoffen der Formel (III) seien solche hervorgehoben, die unter die Formel $H_2N$-($C_2$-$C_{12}$-Alkylen-$NH)_m$-H (IV), bevorzugt die Formel $H_2N$-($C_2$-$C_6$-Alkylen-$NH)_m$-H (V) und besonders bevorzugt die Formel $H_2N$-($C_2$-$C_4$-Alkylen-$NH)_m$-H (VI) fallen. Solche Stoffe sind Diamine und Oligoamine, die sowohl einzeln, als auch als Gemisch, insbesondere als Gemisch der homologen Reihe, in der stets die gleiche Alkylengruppe, aber verschiedene Werte für m auftreten, eingesetzt werden können. Solche Gemische können in gereinigter oder in roher Form und beispielsweise auch als Destillationssumpf aus der Gewinnung solcher Di- und Oligoamine eingesetzt werden. Der Index m bedeutet hierbei eine Ziffer von 1 bis 8, bevorzugt 1 bis 6, besonders bevorzugt 1 bis 4.

Die Verbindung der Formel (I) wird in einer solchen Menge zugesetzt, die ausreicht, die Phthalsäure und die Phthalsäurederivate, wie Phthalsäureanhydrid, Phthalsäure-mono-tolylester und andere, in dem Maße zu binden, daß eine Verunreinigung der o-Tolylsäure mit einem dieser Produkte in der gewünschten Weise unterbleibt. Hierzu werden 1 bis 15, bevorzugt 1,1 bis 10 $NH_2$-Äquivalente der Verbindung der Formel (I), bezogen auf die molare Menge der vorhandenen Phthalsäure und Phthalsäurederivate, zugegeben.

Der Druck zur Entnahme der die reine o-Tolylsäure enthaltenden Destillatfraktion wird erfindungsgemäß auf 70 bis 300 mbar, bevorzugt 80 bis 200 mbar, besonders bevorzugt 100 bis 150 mbar eingestellt.

Die Verbindung der Formel (I) muß nicht notwendigerweise unmittelbar vor Einstellung des Druckes auf einen Wert von 70 bis 300 mbar, d.h. nicht notwendigerweise unmittelbar vor der Entnahme der die reine o-Tolylsäure enthaltenden Destillatfraktion zugefügt werden. Sie kann prinzipiell zu jedem beliebigen Zeitpunkt davor zugesetzt werden. Bevorzugt erfolgt die Zugabe jedoch dann, wenn die Vorlauffraktionen vollständig abdestilliert sind.

In einer dem Fachmann geläufigen Weise wird ein solcher Vorlauf in einer oder mehreren Fraktionen vor der erfindungsgemäßen Gewinnung der o-Tolylsäure entnommen. Es wurde nun weiter gefunden und ist daher eine bevorzugte Variante des erfindungsgemäßen Verfahrens, daß zur Steigerung von Reinheit und Ausbeute der o-Tolylsäure und der zurückzuführenden Stoffe die Sumpftemperatur während der Vorlaufdestillation bis auf höchstens 170°C, bevorzugt höchstens 160°C, besonders bevorzugt höchstens 150°C gesteigert wird. Hierzu ist es vielfach nötig, den Druck beispielsweise bis zu einem Bereich von 10 bis 40 mbar abzusenken. Nach der Entnahme des Vorlaufs in einer oder mehreren Fraktionen und eventuell nach Entnahme einer Zwischenfraktion wird der Druck angehoben und in den für die Gewinnung der reinen o-Tolylsäure erfindungsgemäßen Bereich gebracht.

Es wurde weiterhin gefunden und stellt eine weitere bevorzugte Variante des erfindungsgemäßen Verfahrens dar, daß die Abnahme des Vorlaufs in Gegenwart einer basischen Verbindung durchgeführt wird. Als eine solche basische Verbindung kommt eine aus der Gruppe Ammoniak, Amine, Metallhydroxide und Metallcarbonate in Betracht. Ammoniak und primäre Amine fallen ebenfalls unter die Formeln (I), (II) bzw. (III), so daß die basische Verbindung zur Entnahme des Vorlaufs und die Verbindung der Formeln (I), (II) bzw. (III) für die Gewinnung der reinen o-Tolylsäure als Summe gemeinsam zugegeben werden können und sogar als Stoff identisch sein können. Bevorzugt werden jedoch als basische Verbindungen sekundäre und/oder tertiäre Amine und/oder Metallhydroxide oder Gemische von ihnen zugegeben, wobei die Substituenten der Amine geradkettiges oder verzweigtes $C_1$-$C_8$-Alkyl oder Benzyl sein können und ein sekundäres oder tertiäres Amin auch verschiedene Substituenten tragen kann und wobei die Metalle Natrium, Kalium oder Calcium sind. Als Beispiele seien genannt: Triethylamin, Tributylamin, Dibenzylamin, Tribenzylamin, Tris-(2-hydroxypropyl)-amin, Natriumhydroxid, Kaliumhydroxid und/oder Calciumhydroxid. Die Menge der zugesetzten basischen Verbindung liegt bei 0,1 bis 10 Mol-%, bevorzugt 0,5 bis 5 Mol-%, bezogen auf die vorhandene o-Tolylsäure.

Das erfindungsgemäße Verfahren zur destillativen Gewinnung reiner o-Tolylsäure kann kontinuierlich oder diskontinuierlich durchgeführt werden. Das Verfahren kann auch dahingehend abgeändert werden, daß das im erfindungsgemäßen Verfahren einsetzbare Gemisch mehrmals destilliert wird, wobei die im erfindungsgemäßen Verfahren notwendigen Maßnahmen gegebenenfalls auf die verschiedenen Destillationen verteilt werden. So kann beispielsweise das Einsatzgemisch, gegebenenfalls unter Zusatz der genannten Mengen einer basischen Verbindung zur Bindung stark sauer Protonen, zunächst unter vermindertem Druck vollständig über eine einfache Destillationseinrichtung destilliert werden. Durch diese Maßnahme erreicht man vorweg eine Abtrennung von Hochsiedern und eine Abtrennung von Komponenten, die zu einer Gelbfärbung des Endproduktes führen können. Das Destillat wird anschließend nach dem erfindungsgemäßen und oben beschriebenen Verfahren rektifiziert.

Das erfindungsgemäße Verfahren hat folgende Vorteile:

1. o-Tolylsäure kann aus einem Rohgemisch der o-Xyloloxidation in sehr hoher Ausbeute isoliert werden.

2. Die o-Tolylsäure hat einen Gehalt von über 99 %.

3. Das Verfahren läßt sich kontinuierlich durchführen.

4. Die teiloxidierten Verbindungen (Tolylalkohol und Tolylaldehyd) können in hohen Ausbeuten zur Recyclisierung gewonnen werden.

5. Es fallen keine die Umwelt belastenden Abwässer an.

Nach dem Stand der Technik war nicht vorherzusehen, daß die o-Tolylsäure aus dem komplexen Reaktionsgemisch der o-Xyloloxidation in so hoher Ausbeute und Reinheit herausdestilliert werden könnte. Insbesondere war nicht vorherzusehen, daß die Gewinnung einer o-Tolylsäure mit einem Gehalt von über 99 % nur gelingt, wenn die o-Tolylsäurefraktion in einem begrenzten Druckbereich entnommen wird und vor der Entnahme dieser Fraktion eine Verbindung der Formel (I) mit einer $H_2N$-Gruppe dem Destillationssumpf zugesetzt wird. Wird der erfindungsgemäße Druckbereich unterschritten, was bei der Destillation so hochsiedender Verbindungen, wie der o-Tolylsäure, normalerweise eine Notwendigkeit ist, und wird keine Verbindung der Formel (I) zugesetzt, gelingt eine Reinisolierung der o-Tolylsäure nicht, wie ein Vergleichsbeispiel zeigt, bei welchem das Reaktionsgemisch der o-Xyloloxidation ohne Zusatz einer Verbindung der Formel (I) bei einem Druck von 40 mbar destilliert wurde. Es ist auch als überraschend zu bezeichnen, daß die o-Tolylsäure trotz der notwendigen hohen Destillationstemperaturen keine nennenswerten Zersetzungs- oder Umwandlungsreaktionen zeigt.

Beispiel 1

Bei der Destillation wurde ein Produktgemisch eingesetzt, das bei der Oxidation von o-Xylol mit Luft in Gegenwart von Kobaltsalzen höherer Fettsäuren anfällt. Die Destillation erfolgte über eine 1 m x 2,5 cm Spiegelmantelkolonne, gefüllt mit Maschendrahtgewebe aus V2A-Stahl (4 x 4 mm, 3600 Maschen/cm²). Das Rücklaufverhältnis betrug 10:1. Die Zusammensetzung des Ausgangsgemisches sowie das Ergebnis der Destillation ist in der Tabelle 1 zusammengestellt. Den Gehaltsangaben liegen Gaschromatographie(GC)- und Hochdruckflüssigkeitschromatographie(HPLC)-Bestimmungen zugrunde. Dem Einsatzgemisch wurde zu Beginn der Destillation 15 g Tribenzylamin und nach Abnahme der Fraktion 3 20 g Ethylendiamin zugesetzt. 73,4 % der im Einsatz vorhandenen o-Tolylsäure liegen in der Hauptfraktion (Fraktion 6) mit einem Gehalt von 99,3 % vor. Zusätzliche 21,3 % der o-Tolylsäure sind in Zwischenfraktionen (Fraktion 4, 5, 7 und Kolonneninhalt) enthalten, die sich bei der Destillation recyclisieren lassen. In den Vorfraktionen 1 bis 3, die zur Oxidation zurückgeführt werden, befinden sich neben o-Xylol auch o-Tolylaldehyd und o-Tolylalkohol. Insgesamt lassen sich 93,2 % des o-Tolylaldehyds und 91,7 % des o-Tolylalkohols, jeweils bezogen auf die im Einsatz vorhandenen Mengen, recyclisieren. Alle weiteren Angaben stehen in Tabelle 1.

Beispiel 2 (Vergleichsbeispiel)

Es wurde wie im Beispiel 1 gearbeitet, jedoch mit dem Unterschied, daß ab Fraktion 2 bei einem Druck von 40 mbar gearbeitet wurde, daß vor Entnahme der o-Tolylsäure keine Verbindung der Formel $H_2N-R^2$ zugesetzt wurde und daß zu Beginn der Destillation keine Base zugesetzt wurde. Alle weiteren Angaben stehen in Tabelle 2.

Tabelle 1

| | Druck [mbar] | Sdp. [°C] | Sumpftemperatur [°C] | Menge [g] | Gehalt % | | | |
|---|---|---|---|---|---|---|---|---|
| zum Beispiel 1 | | | | | | | | |
| | | | | | o-Xylol | o-Tolylaldehyd | o-Tolylalkohol | o-Tolylsäure |
| Einsatz | | | | 2927 | 62,8 | 2,6 | 2,1 | 24,7 |
| Fraktion 1 | 60 | 60-61 | 79-130 | 1716,0 | 99,2 | - | - | - |
| Fraktion 2 | 40 | 30 | 127-138 | 104,3 | 94,3 | 3,3 | - | - |
| Fraktion 3 | 20 | 82-108 | 138-158 | 127,1 | - | 53,1 | 32,4 | 0,9 |
| Fraktion 4 | 20 | 108-144 | 158-159 | 45,0 | - | - | 29,9 | 58,7 |
| Fraktion 5 | 120 | 152-189 | 193-197 | 44,6 | - | - | 3,9 | 95,4 |
| Fraktion 6 | 120 | 189 | 197-230 | 534,4 | - | - | - | 99,3 |
| Fraktion 7 | 40 | 162 | 198-233 | 57,6 | - | - | - | 93,2 |
| Kolonneninhalt | | | | 35,5 | - | - | - | 89,4 |

Tabelle 2

| | Druck [mbar] | Sdp. [°C] | Menge [g] | Gehalt % | | | |
|---|---|---|---|---|---|---|---|
| zum Beispiel 2 | | | | | | | |
| | | | | o-Xylol | o-Tolylaldehyd | o-Tolylalkohol | o-Tolylsäure |
| Einsatz | | | 1777,1 | 62,8 | 2,6 | 2,1 | 24,7 |
| Fraktion 1 | 60 | 60- 61 | 993,8 | 99,3 | - | - | - |
| Fraktion 2 | 40 | 30- 66 | 121,4 | 92,8 | 5,1 | - | - |
| Fraktion 3 | 40 | 66-158 | 75,5 | 6,4 | 49,6 | 25,3 | 13,4 |
| Fraktion 4 | 40 | 158-160 | 41,2 | - | 0,4 | 7,8 | 81,9 |
| Fraktion 5 | 40 | 160-161 | 102,7 | - | - | 5,7 | 89,3 |
| Fraktion 6 | 40 | 161-162 | 136,2 | - | - | - | 94,1 |
| Fraktion 7 | 40 | 162 | 89,0 | - | - | - | 93,3 |
| Fraktion 8 | 40 | 162 | 27,9 | - | - | - | 86,2 |
| Kolonneninhalt | | | 34,8 | - | - | - | 82,9 |

## Ansprüche

1. Verfahren zur destillativen Gewinnung von o-Tolylsäure aus den bei der Sauerstoffoxidation von o-Xylol anfallenden Reaktionsgemischen, dadurch gekennzeichnet, daß die die reine o-Tolylsäure enthaltende Destillatfraktion in einem Druckbereich von 70 bis 300 mbar entnommen wird und dem Destillationssumpf vorher 1 bis 15 $NH_2$-Äquivalente pro Mol im Destillationssumpf vorhandener Phthalsäure oder Phthalsäurederivate einer oder mehrerer Verbindungen der Formel
$H_2N-R^1$
zugesetzt wurde, worin
$R^1$ für H, Cl, Br, OH, CN, $NH_2$, $CONH_2$, $NHCONH_2$, $R^2$, $NHR^2$, $OR^2$, $COR^2$, $SO_2R^2$ oder $CONHR^2$ steht, wobei
$R^2$ geradkettiges oder verzweigtes $C_1$-$C_{30}$-Alkyl, $C_6$-$C_{12}$-Aryl oder $C_7$-$C_{10}$-Aralkyl bedeutet und wobei $R^2$ durch COOH, $C_1$-$C_4$-Alkoxy, OH und/oder $NH(C_2$-$C_{12}$-Alkylen-NH$)_n$-H substituiert sein kann, worin n eine Ziffer von 0 bis 8 bedeutet.

2. Verfahren nach Anspruch 1, dadurch gekennzeichnet, daß Reaktionsgemische eingesetzt werden, welche bei der Sauerstoffoxidation von o-Xylol in flüssiger Phase mit oder ohne Hinzuziehung eines Lösungs-oder Verdünnungsmittels anfallen.

3. Verfahren nach Anspruch 2, dadurch gekennzeichnet, daß Reaktionsgemische eingesetzt werden, bei denen die Oxidation in Gegenwart von Schwermetallverbindungen mit oder ohne Aktivatoren durchgeführt wurde.

4. Verfahren nach Anspruch 1, dadurch gekennzeichnet, daß man in Gegenwart einer Verbindung der Formel
$H_2N-R^{11}$
arbeitet, in denen
$R^{11}$ für H, OH, $NH_2$, $CONH_2$, $NHCONH_2$, $R^2$, $COR^2$ oder $CONHR^2$ steht, wobei
$R^2$ geradkettiges oder verzweigtes $C_1$-$C_{30}$-Alkyl, $C_6$-$C_{12}$-Aryl oder $C_7$-$C_{10}$-Aralkyl bedeutet und wobei $R^2$ durch COOH, $C_1$-$C_4$-Alkoxy, OH und/oder $NH(C_2$-$C_{12}$-Alkylen-NH$)_n$-H substituiert sein kann, worin n' eine Ziffer von 0 bis 8 bedeutet.

5. Verfahren nach Anspruch 4, dadurch gekennzeichnet, daß man in Gegenwart einer Verbindung der Formel
$H_2N-R^{21}$
arbeitet, in der
$R^{21}$ für Wasserstoff, geradkettiges oder verzweigtes $C_1$-$C_{20}$-Alkyl, Phenyl, Benzyl oder -$(C_2$-$C_{12}$-Alkylen-NH$)_n$-H steht, worin n eine Ziffer von 1 bis 8 bedeutet, und wobei $C_1$-$C_{20}$-Alkyl, Phenyl und Benzyl durch

7

COOH, $C_1$-$C_4$-Alkoxy und/oder OH substituiert sein können.

6. Verfahren nach Anspruch 5, dadurch gekennzeichnet, daß Di- und Oligoamine oder Gemische eingesetzt werden, die unter die Formel $H_2N$-$(C_2$-$C_{12}$-Alkylen-NH$)_m$-H, bevorzugt unter die Formel $H_2N(C_2$-$C_6$-Alkylen-NH$)_m$-H, besonders bevorzugt unter die Formel $H_2N$-$(C_2$-$C_4$-Alkylen-NH$)_m$-H fallen, in denen m eine Ziffer von 1 bis 8, bevorzugt 1 bis 6, besonders bevorzugt 1 bis 4 bedeutet.

7. Verfahren nach Anspruch 1, dadurch gekennzeichnet, daß ein Vorlauf in einer oder mehreren Fraktionen vor der die reine o-Tolylsäure enthaltenden Destillatfraktion bei einer Sumpftemperatur von höchstens 170° C, bevorzugt höchstens 160° C, besonders bevorzugt höchstens 150° C entnommen wird.

8. Verfahren nach Anspruch 7, dadurch gekennzeichnet, daß vor der Entnahme des Vorlaufs in einer oder mehreren Fraktionen dem Destillationssumpf eine basische Verbindung aus der Gruppe von Ammoniak, Aminen, Metallhydroxiden oder Metallcarbonaten zugesetzt wird.

9. Verfahren nach Anspruch 8, dadurch gekennzeichnet, daß als basische Verbindung sekundäre und/oder tertiäre Amine und/oder Metallhydroxide oder ein Gemisch von ihnen zugesetzt werden.

10. Verfahren nach Anspruch 8, dadurch gekennzeichnet, daß die basische Verbindung in einer Menge von 0,1 bis 10 Mol-%, bevorzugt 0,5 bis 5 Mol-%, bezogen auf vorhandene o-Tolylsäure, zugesetzt wird.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | PATENT ABSTRACTS OF JAPAN, Band 12, Nr. 398 (C-538)[3245], 21. Oktober 1988; & JP-A-63 141 945 (MITSUBISHI GAS CHEM. CO. INC.) 14-06-1988 * Zusammenfassung * --- | 1-5,7-10 | C 07 C 63/04 C 07 C 51/487 C 07 C 51/44 |
| Y | EP-A-0 163 212 (BAYER) * Ansprüche 1-3,6; Seite 6, Zeilen 7-21 * --- | 1-5 | |
| Y | DE-C-2 636 489 (BAYER) * Anspruch; Spalte 2, Zeilen 5-13 * ----- | 1-5 | |

RECHERCHIERTE SACHGEBIETE (Int. Cl.5)

C 07 C 63/00
C 07 C 51/00

Der vorliegende Recherchenbericht wurde für alle Patentansprüche erstellt

| Recherchenort | Abschlußdatum der Recherche | Prüfer |
|---|---|---|
| DEN HAAG | 13-10-1989 | KLAG M.J. |